Europäisches Patentamt

European Patent Office

Office européen des brevets

(19)

(11) Veröffentlichungsnummer: **0 510 375 A1**

# (12) EUROPÄISCHE PATENTANMELDUNG

(21) Anmeldenummer: **92105252.8**

(22) Anmeldetag: **27.03.92**

(51) Int. Cl.5: **C07C 53/06**

(30) Priorität: **25.04.91 DE 4113470**

(43) Veröffentlichungstag der Anmeldung:
**28.10.92 Patentblatt 92/44**

(84) Benannte Vertragsstaaten:
**BE DE IT NL**

(71) Anmelder: **DEGUSSA AG**
**Weissfrauenstrasse 9**
**W-6000 Frankfurt (Main)(DE)**

(72) Erfinder: **Werle, Peter, Dr.**
**Im Börner 43**
**W-6460 Gelnhausen(DE)**
Erfinder: **Trageser, Martin**
**Leipziger Strasse 14**
**W-6460 Gelnhausen-Hoechst(DE)**
Erfinder: **Duderstadt, Ulrike**
**Berliner Strasse 18**
**W-6467 Hasselroth 1(DE)**

(54) **Verfahren zur Herstellung von Calciumformiat.**

(57) Erfindungsgemäß erfolgt die Herstellung von Calciumformiat in wäßriger Phase durch Umsetzung von Calciumhydroxid mit Formaldehyd in Gegenwart von Wasserstoffperoxid oder durch Umsetzung von Calciumperoxid mit Formaldehyd;
das Einsatz-Molverhältnis von CaO beziehungsweise Ca(OH)$_2$ zu H$_2$CO zu H$_2$O$_2$ beträgt 1 zu 2 zu 1 bis 1,2, dasjenige von CaO$_2$ zu H$_2$CO 1 zu 2. Calciumformiat wird in hoher Reinheit in nahezu quantitativer Ausbeute gewonnen; das Verfahren vermeidet die Nachteile der vorbekannten Herstellung von Calciumformiat aus Calciumhydroxid und Formaldehyd.

EP 0 510 375 A1

Die Erfindung betrifft ein Verfahren zur Herstellung von Calciumformiat in wäßriger Phase durch Umsetzung von Formaldehyd mit Calciumhydroxid in Gegenwart von Wasserstoffperoxid oder durch Umsetzung von Formaldehyd mit Calciumperoxid.

Calciumformiat, das Calciumsalz der Ameisensäure, wird praktisch ausschließlich als Nebenprodukt bei der Herstellung der mehrwertigen Alkohole (Polyole) Pentaerythrit, Trimethylolethan, Trimethylolpropan und Neopentylglykol gewonnen. Es bildet sich bei der Umsetzung der durch Aldolkondensation zugänglichen 3-Hydroxyaldehyde mit Formaldehyd in Gegenwart von Calciumhydroxid; Calciumformiat ist somit das Oxidationsprodukt einer gemischten Cannizzaro-Reaktion. Auch Natriumformiat wird als Nebenprodukt der Polyolherstellung gewonnen.

Calciumformiat wird für vielfältige Zwecke eingesetzt, wie beispielsweise als Hilfsmittel in der Gerberei, zur Erzeugung von Ameisensäure, als Abbindebeschleuniger in der Zementindustrie, als Silierhilfsmittel sowie in steigendem Maß als nutritiv wirksames Futtermitteladditiv in der Tierernährung.

Das letztgenannte Einsatzgebiet - Calciumformiat dient zur Verbesserung der Futterverwertung, zur Reduzierung von Verdauungsstörungen, Vermeidung von mikrobiellem Futterverderb (siehe Broschüre der Degussa AG. "Calciumformiat" - Ch 609-1-105-988 DD) - stellt besondere Qualitätsanforderungen, damit das Calciumformiat enthaltende Futter von den Tieren nicht verweigert wird.

Nachteilig an der Herstellung von Calciumformiat als Nebenprodukt der Polyolsynthese ist, daß eine Erhöhung der Formiatproduktion nur zusammen mit einer Erhöhung der betreffenden Polyolproduktion erreicht werden kann; dies setzt entsprechende Absatzmöglichkeiten des Polyols voraus.

Bekannt sind auch Verfahren zur Herstellung von Calciumformiat durch Carbonylierung des Calciumhydroxids (vgl. beispielsweise Gmelin-Handbuch, Band Ca(B), Seiten 161-162 sowie Ullmann's Encyclopedia, 4th ed., Vol. A 12, Seiten 23-24). Diese Verfahren erfordern im allgemeinen technisch aufwendige Anlagen, hohe Drucke, hohe Temperaturen und meist lange Reaktionszeiten.

Die Cannizzaro-Reaktion von Formaldehyd mit starken anorganischen Basen wird durch folgendes Schema wiedergegeben:

$$Me\,(OH)_n\ +\ 2nCH_2O \rightarrow Me(OOCH)_n\ +\ nCH_3OH$$

n = 1 für Me = K, Na
n = 2 für Me = Ca, Ba

Hierbei ist der Verlauf dieser Reaktion vom verwendeten Metallhydroxid abhängig. Zahlreiche Untersuchungen haben gezeigt, daß gewisse Metallhydroxide nicht die Cannizzaro-Reaktion begünstigen, sondern eine Autokondensation des Formaldehyds. Obwohl das Formaldehyd-Molekül kein $\alpha$-H-Atom besitzt und deshalb keine normale Aldolkondensation eingehen kann, werden Hydroxyaldehyde und Ketone, besonders Pentosen und Hexosen, die sogenannten Formosen, durch eine praktisch analoge Reaktion gebildet [T. Mizuno, A. Weiss, Adv. Carbohyd. Chem. Biochem. 29, 173 (1974)]. Zu den formosebildenden Hydroxiden gehören insbesondere $Pb(OH)_2$, $Sn(OH)_2$, $TlOH$ und $\overline{Ca}(OH)_2$.

Bisher war es daher nicht möglich, durch Umsetzung von beispielsweise Kalkmilch mit Formaldehyd gemäß nachfolgender Gleichung

$$Ca(OH)_2\ +\ 4\,CH_2O \rightarrow Ca(OOCH)_2\ +\ 2\,CH_3OH$$

Calciumformiat in hohen Ausbeuten zu isolieren, da die gleichzeitig einsetzende autokatalytische Formaldehydkondensation nach einer bestimmten, von der jeweiligen Reaktionstemperatur und ggf. anwesenden Begleitstoffen abhängenden Inkubationszeit noch vorhandenen Formaldehyd praktisch augenblicklich in stark exothermer Reaktion in Formosen überführt und somit der Formiatbildung entzieht.

Die sowjetische Patentschrift 1474157 beschreibt ein Verfahren zur Herstellung von Calciumformiat durch Cannizzaro-Reaktion aus Calciumhydroxid und Formaldehyd, bei dem die Verzuckerung des Formaldehyds durch den Zusatz der Metallsalze $MnSO_4$, $FeSO_4$, $Cu_2Cl_2$ oder $Ce(NO_3)_2$ weitgehend verhindert wird. Die Ausbeuten an Calciumformiat sind hier zwar deutlich besser als beim Arbeiten ohne Inhibitoren, sie erreichen beim Nacharbeiten des besten Beispiels (Nr. 6) etwa 89 %. Nachteilig sind aber die sehr langen Reaktionszeiten (5 h), die niedrige Raum-Zeit-Ausbeuten bedingen, sowie die hohen Verdünnungen, die das kostenintensive Verdampfen großer Wassermengen zur Folge haben. Außerdem muß der in der Lösung verbleibende Formaldehyd (in Beispienl 6 wurden etwa 8 % der eingesetzten Menge nicht umgesetzt) vom Calciumformiat abgetrennt werden; im allgemeinen ist dazu wegen der besonderen Eigenschaften des Formaldehyds eine Destillation unter Druck notwendig. Diese nicht vollständige Umsetzung macht die an und für sich technisch wenig aufwendige Cannizzaro-Reaktion für die großtechnische Calciumformiatsynthese in dieser Form nicht verwendbar, da das Problem des überschüssigen Formaldehyds nicht gelöst ist und Calciumformiat aus solchen Lösungen nicht ausreichend formaldehydfrei auskristallisiert werden kann. Reaktionsbedingt fallen zudem pro Mol Calciumformiat zwei Mol Methanol an, was einerseits zu einem hohen Formaldehydeinsatz und andererseits zu Aufwendungen zur Abtrennung des Methanols aus dem Reaktionsgemisch führt.

Aufgabe der Erfindung ist somit, das aus der SU-PS 1474157 bekannte Verfahren so zu verbessern, daß Calciumformiat ohne die aufgezeigten Nachteile in wirtschaftlicher Weise hergestellt werden kann.

Die Aufgabe wird gelöst durch ein Verfahren zur Herstellung von Calciumformiat durch Umsetzung von Formaldehyd mit einer Calciumverbindung in wäßriger Phase bei einer Temperatur im Bereich von 20 bis 90 °C und Isolierung des Formiats aus der wäßrigen Phase,

dadurch gekennzeichnet,

daß man die Umsetzung in Gegenwart von Wasserstoffperoxid und/oder Calciumperoxid durchführt, wobei als Reaktionspartner für die Umsetzung Calciumoxid oder Calciumhydroxid, Formaldehyd und Wasserstoffperoxid im Molverhältnis von 1 zu 2 zu 1 bis 1,2 oder Calciumperoxid und Formaldehyd im Molverhältnis von 1 zu 2 eingesetzt werden.

Das erfindungsgemäße Verfahren beruht auf folgenden Reaktionsgleichungen

$$(1) \quad Ca(OH)_2 + 2H_2CO + H_2O_2 \rightarrow Ca(HCOO)_2 + H_2 + 2H_2O$$

$$(2) \quad CaO_2 + 2H_2CO \rightarrow Ca(HCOO)_2 + H_2$$

Das Peroxid gemäß Gleichung (2) unterliegt in der wäßrigen Phase der Hydrolyse, wobei $H_2O_2$ und Ca(OH)$_2$ gebildet werden, so daß die Reaktionen nach (1) und (2) auch parallel nebeneinander ablaufen können. Für die Praxis kann Gleichung (2) zur Herstellung von Calciumformiat Bedeutung erlangen, da sich Calciumperoxid auch in-situ aus Ca(OH)$_2$ und $H_2O_2$ bilden kann.

Die Umsetzung wird im Temperaturbereich von 20 °C bis 90 °C durchgeführt. Vorzugsweise wird zwischen 30 °C und 70 °C, insbesondere 40 °C und 60 °C, gearbeitet. Durch Begrenzung der Temperatur durch Kühlen der exothermen Reaktion läßt sich die Bildung von sogenannten Formosen aus der Formaldehyd-Verzuckerung minimieren.

Zur Durchführung der Umsetzung gemäß Gleichung (1) können in die wäßrige Lösung oder Suspension des Calciumhydroxids eine wäßrige Formaldehydlösung und eine wäßrige Wasserstoffperoxidlösung simultan zugegeben werden; während der Zugabe wird gut gemischt, etwa durch Rühren oder Umpumpen des Reaktionsgemischs. Nach beendeter Zugabe der Reaktionspartner läßt man, soweit noch erforderlich, nachreagieren - das Reaktionsende läßt sich durch Bestimmung des Formaldehydgehalts einfach ermitteln. Gemäß einer besonders bevorzugten Ausführungsform werden Calciumhydroxid und Formaldehyd in wäßriger Phase vorgelegt und die Wasserstoffperoxidlösung zudosiert. Die Zugabe eines Gemischs aus wäßrigem Formaldehyd und Wasserstoffperoxid zu einer wäßrigen Ca(OH)$_2$-Suspension oder das Eintragen von Ca(OH)$_2$ in ein Gemisch aus wäßrigem Formaldehyd und Wasserstoffperoxid werden weniger bevorzugt.

Sofern das erfindungsgemäße Verfahren gemäß Gleichung (2) durchgeführt wird, so wird zweckmäßigerweise Calciumperoxid in eine wäßrige Formaldehydlösung eingetragen.

Die Bildung von Formosen, welche durch Ca(OH)$_2$ katalysiert wird und damit die Herstellung von Calciumformiat beeinträchtigen kann, läßt sich weitgehend vermeiden, wenn die Umsetzung zusätzlich in Gegenwart geeigneter Inhibitoren durchgeführt wird. Bei diesen Inhibitoren, welche die gewünschte Umsetzung nicht beeinträchtigen und die Gewinnung reiner Formiate nicht erschweren sollen, handelt es sich um unterschiedliche Metallverbindungen, teilweise auch um Metalle in elementarer Form, welche im allgemeinen in einer Menge im Bereich von 0,1 mMol bis 10 mMol pro Mol Formaldehyd dem Reaktionsgemisch vor der eigentlichen Umsetzung zugesetzt werden.

Über die Wirksamkeit der Metallsalze als Inhibitoren kann sich der Fachmann durch einen einfachen Vorversuch einen raschen Einblick verschaffen: Eine Mischung aus Ca(OH)$_2$ und wäßrigem Formaldehyd (37 Gew.-%, unstabilisiert) - Molverhältnis 1:2 - wird in Gegenwart des Metallsalzes (z. B. 1 mMol pro Mol Formaldehyd) bei 70 °C gerührt; eine nach einer bestimmten Reaktionsdauer auftretende plötzliche Gelbfärbung zeigt an, daß Formaldehyd durch Formosenbildung verbraucht wird. Die folgende Tabelle gibt eine Übersicht über die inhibierende Wirkung verschiedener Metallverbindungen.

Aus der Tabelle ist zu entnehmen, daß Elemente einer Gruppe des Periodensystems sehr unterschiedliche Aktivitäten aufweisen. So ist z. B. $CrO_4^{2-}$ praktisch wirkungslos, während $WO_4^{2-}$ einen sehr effektiven Inhibitor darstellt; analog ist $TiCl_4$ kaum wirksam, während $ZrCl_4$ zu den wirksamsten Katalysatoren zählt - in dem alkalischen Reaktionsmilieu werden hier Oxidhydrate vorliegen. Noch überraschender ist, daß auch

3

Unterschiede bei einem Element auftreten, das an unterschiedliche Anionen gebunden ist. Besonders ausgeprägt ist dieser Effekt beim Mangan. Mn$^{II}$-acetat zeigt überragende Inhibitoreigenschaften, obwohl man annehmen sollte, daß im vorliegenden alkalischen Medium unabhängig von der Wahl des Mangansalzes einheitlich Mn-hydroxide vorliegen.

Tabelle: Aktivitäten verschiedener Inhibitoren zur Inhibierung der Formosen-Bildung

| Inhibitor | Stabilisierung (Min) | Inhibitor | Stabilisierung (Min) |
|---|---|---|---|
| | | $Mn_2O_3$ | 14 |
| ohne | 13 | $MnSO_4$ | 29 |
| | | $MnSO_4$ (3,8) | 21 |
| LiBr | 13 | $MnCl_2$ | 45 |
| $KJO_4$ | 13 | Mn(II)-acetat | 90 |
| KSeCN | 13 | Mn(III)-acetat | 30 |
| $SbCl_3$ | 14 | Mn(II)-acetyl-acetonat | 45 |
| $Bi(NO_3)_2$ | 15 | | |
| $B_2O_3$ | 26 | $FeSO_4$ | 20 |
| $NaBO_2$ | 36 | $FeCl_3$ (3,8) | 20 |
| $AlCl_3$ | 25 | Co(III)-acetat | 15 |
| $Al_2(SO_4)_3$ | 20 | $NiSO_4$ | 14 |

| | | | |
|---|---|---|---|
| $TiCl_4$ | 16 | | |
| $ZrCl_4$ | >90 | Cu pulver (3,8) | 18 |
| $ZrCl_4$ (3,8) | >90 | $CuSO_4$ | 17 |
| $HfCl_4$ | 35 | $CuCl_2$ (3,8) | 21 |
| | | $Cu(NO_3)_2$ (3,8) | 26 |
| $V_2O_5$ | 14 | $AgNO_3$ | 14 |
| $VOSO_4$ | 20 | | |
| $NbCl_5$ | 20 | | |
| $K_2CrO_4$ | 15 | | |
| $Na_2MO_4$ | 24 | | |
| $Na_2WO_4$ | 51 | | |
| $WCl_4$ | 15 | | |

Testbedingungen: 15,2 g $Ca(OH)_2$ (97 %) + 32,5 g $CH_2O$ (37 Gew.-%); T=70 °C; U=200/Min.

Inhibitoreinsatz: 7,6 mg, bezogen auf Zentralatom; Versuche mit der halben Inhibitor- menge (3,8 mg) wurden in der Tabelle gekennzeichnet.

Als Inhibitor sind Salze aus der Reihe $CuCl_2$, $Cu(NO_3)_2$; $MnCl_2$, $MnSO_4$, Mn(II)-acetat, Mn(II)-acetylacetonat, Mn(III)-acetat; Molybdate und insbesondere Wolframate der Alkali- und Erdalkalimetalle, $ZrCl_4$, $ZrOCl_2$, $HfCl_4$, Borate der Alkali- und Erdalkalimetalle sowie $AlCl_3$ gut geeignet. Eine herausragende Wirkung zeigen Mangansalze, Zirkonhalogenide und Wolframate. Von den hochwirksamen Inhibitoren sind Zugabemengen im Bereich von 0,1 bis 2 mMol pro Mol Formaldehyd im allgemeinen ausreichend. Falls erforderlich, kann die Umsetzung auch in Gegenwart einer geringen Menge Entschäumer, wie z. B. Produkten auf Siliconbasis, durchgeführt werden, um ein Schäumen der Reaktionsmischung zu verhindern.

Die Umsetzung des Calciumhydroxids mit Formaldehyd und Wasserstoffperoxid sowie jene zwischen Calciumperoxid und Formaldehyd liefert praktisch wasserklare Formiatlösungen, die freien Formaldehyd nur noch in Spuren enthalten, weil der Formaldehydumsatz in der Regel über 95 %, unter optimierten Bedingungen meist über 98 % beträgt. Vor der Isolierung des Formiats aus der wäßrigen Reaktionslösung wird, sofern erforderlich, der pH-Wert durch Zugabe von Ameisensäure auf 6,5 bis 7,5 eingestellt. Sollte aufgrund von Nebenbestandteilen im eingesetzten Oxid, Hydroxid bzw. Peroxid die Lösung noch Trübstoffe enthalten, dies dürfte beim Einsatz von Kalk zur Herstellung von Calciumformiat meist der Fall sein, werden diese mit oder ohne Filterhilfsmittel in üblicher Weise abgetrennt. Die Isolierung des Formiats aus der klaren Lösung erfolgt in an sich bekannter Weise, etwa durch Verdampfungskristallisation, Abtrennung der Kristalle von der Mutterlauge und Trocknung der Kristalle.

Im erfindungsgemäßen Verfahren wird die Gesamtmenge an Wasser möglichst so niedrig gehalten, daß die erhaltene Formiatlösung möglichst hochkonzentriert ist und der Energieaufwand bei der Isolierung des Formiats gering ist. Formaldehyd wird im allgemeinen als handelsübliche wäßrige Lösung, insbesondere mit einem Gehalt von 37 Gew.-%, eingesetzt; im Prinzip können auch Formaldehyd-Oligomere der Formel $HO-(CH_2O)_nH$ mit n = 10 bis 30 eingesetzt werden, sofern vor der eigentlichen Umsetzung depolymerisiert wird. Wäßriges Wasserstoffperoxid kann in beliebiger Konzentration, zweckmäßigerweise in handelsüblicher Konzentration, insbesondere zwischen 30 und 70 Gew.-%, eingesetzt werden. Erfindungsgemäß kann die Gesamtmenge an Wasser auch so bemessen werden, daß die Reaktionslösung nach dem Reaktionsende weniger Wasser enthält als es der Löslichkeit des Calciumformiats entspricht; es liegen hier also metastabile Formiatlösungen vor, wodurch sich der Energieaufwand bei der Isolierung des Formiats weiter verringert.

Das erfindungsgemäße Verfahren erlaubt die Herstellung von Calciumformiat in praktisch quantitativer Ausbeute und zudem in hoher Reinheit und mit hoher Raum-Zeit-Ausbeute. Das Verfahren kann diskontinuierlich oder kontinuierlich durchgeführt werden. Der technische Aufwand ist niedrig, da die Raum-Zeit-Ausbeute hoch ist und die Reaktionslösung nur noch Spuren an nicht umgesetztem Formaldehyd enthalten, so daß sich eine Druckdestillation zur Abtrennung von Formaldehyd erübrigt. Anders als bei der üblichen Cannizzaro-Reaktion sind im erfindungsgemäßen Verfahren pro Mol Ca-Verbindung nur zwei Mol Formaldehyd (anstelle vier Mol in der Cannizzaro-Reaktion) erforderlich, und es entsteht kein Methanol, das folglich auch nicht abdestilliert werden muß. Der bei der Reaktion entstehende Wasserstoff kann ferner verbrannt und somit zur Energiegewinnung und damit Absenkung der Verfahrenskosten eingesetzt werden.

**Beispiel 1**

In einem mit Rührer und Gasableitungsrohr versehenen Reaktor werden 76,4 g Calciumhydroxid (97 gew.-%ig; 1 Mol), 600 ml Wasser, 0,200 g Zirkontetrachlorid [0,00086 Mol] und 162,5 g Formaldehyd (37 gew.-%ig; 2 Mol) bei ca.40 bis 50 °C vorgelegt. Unter guter Kühlung werden innerhalb 30 min 120 g $H_2O_2$ - (30 gew.-%ig; 1,05 Mol) zugetropft. Die Temperatur wird bei max. 50 °C gehalten. Man läßt 15 min nachrühren - der pH-Wert der Lösung fällt auf etwa 7 - 7,5 - und filtriert von den unlöslichen Kalkbestandteilen (Gangart) ab. Erhalten werden 1020 ml (Volumenzunahme durch Waschwasserzusatz) einer klaren, farblosen Lösung.
Die komplexometrische Bestimmung ergibt 126 g Calciumformiat, das sind 97 % d. Th. Der Restformaldehyd liegt bei 0,07 %. Durch Eindampfen der Lösung und Trocknen wird kristallines, farblosen Calciumformiat in einer Reinheit von 99,7 % erhalten (Gehaltsbestimmung durch Abtrennen der Ca-Ionen mittels eines stark sauren Kationenaustauschers und Titration der freigesetzten Ameisensäure).

**Beispiel 2**

Ansatz analog Beispiel 1.
Als Inhibitor werden 0,230 g Mangan-II-acetat $2H_2O$ [0,00086 Mol] eingesetzt.
Aus der schwach bräunlichen Reaktionsmischung werden nach Filtration 1020 ml einer klaren Lösung mit einem pH von 7,6 erhalten. Der Restformaldehyd liegt bei 0,08 %, die Titration ergibt 125 g Calciumformiat (96 % d. Th).

**Beispiel 3**

38,2 g Kalk (97 gew.-%ig) werden in 300 ml Wasser vorgelegt, 0,25 g $NaBO_2$-$4H_2O$ [0,0018 Mol] und 81 g Formaldehyd (37 gew.-%ig) zugegeben. Innerhalb 15 min werden bei maximal bis 60 °C 30 gew.-%iges $H_2O_2$ zugetropft, 30 min nachgerührt und filtriert. Man erhält 475 ml einer farblosen Lösung mit pH 7,2 und einem Restformaldehydgehalt von 0,06 %. Ausbeute an Calciumformiat: 62,0 g ≙ 95.3 % d. Th.

**Beispiel 4**

Es werden 152,8 g Kalk (97 gew.-%ig, 2.0 Mol) in 1200 ml Wasser vorgelegt, innerhalb von ca. 30 min unter Kühlung im Eisbad und Durchleiten von Stickstoff 324,0 g Formaldehyd (37 gew.-%ig, 4,0 Mol) und 240 g $H_2O_2$ (30 gew.-%ig, 2,1 Mol) getrennt zugetropft. Die Temperatur wird auf 50 bis 55 °C begrenzt. Man rührt 30 min bei 40 bis 50 °C nach. Die Lösung mit einem ph von etwa 10 wird durch 2 ml Ameisensäure auf pH 7 eingestellt, etwas Kieselgur zugegeben und filtriert. 1880 ml klare Lösung mit 250 g Calciumformiat. Der Restformaldehydgehalt ist hier etwas höher, er liegt bei 0,1 %.

**Beispiel 5**

150 ml Wasser und 81 g Formaldehyd (37 gew.-%ig, 1,0 Mol) werden bei Raumtemperatur vorgelegt. In diese Lösung trägt man 60,1 g Calciumperoxid (60 gew.-%ig, 0,5 Mol) ein und rührt 2 h bei 40 °C. Zusatz von Entschäumer ist notwendig. Die stark alkalische Suspension wird filtriert und mit 0,7 ml Ameisensäure neutral gestellt. 455 ml klare, farblose Lösung, Formaldehydgehalt <0,1 %.

# EP 0 510 375 A1

**Beispiel 6**

Für die Erzielung eines niedrigen Restformaldehydgehaltes weniger geeignet ist die Zugabe einer Mischung aus Formaldehyd und Wasserstoffperoxid.

Vorlage: 76,4 g Ca(OH)$_2$ (97 gew.-%ig) und 200 ml H$_2$O. Einige Tropfen Entschäumer auf Siliconbasis.

Zulauf: Mischung aus 400 ml H$_2$O; 162 g Formaldehyd (37 gew.-%ig) und 120 g H$_2$O$_2$ (30 gew.-%ig).

Start der Reaktion bei Raumtemperatur und Halten unter Kühlung bei 50 bis 55 °C.

Zulaufdauer: 30 min

Nachreaktion: 30 min

pH der ausreagierten Lösung 12.

Neutralisation durch Zugabe von 3,5 ml Ameisensäure.

Das klare Filtrat enthält noch 0,6 % freien Formaldehyd, der vor Aufarbeitung auf Calciumformiat auf ca. 0,1 % reduziert werden müßte.

**Beispiel 7**

76,4 g Kalk (97 gew.-%ig) werden in 400 ml Wasser vorgelegt, 0,05 g H$_2$WO$_4$ [0,0002 Mol] und 162 g Formaldehyd (37 Gew.-%) zugegeben. Innerhalb 15 min. werden bei maximal 50°C 72 g 50 gew.-%iges H$_2$O$_2$ zugetropft, 30 min. nachgerührt und filtriert.

Man erhält 710 g einer farblosen, an Calciumfomiat übersättigten Lösung mit einem Formaldehydrestgehalt von 0,07 % und einem Calciumformiatgehalt von 17,8 Gew.% (Löslichkeit von Ca-formiat in H$_2$O = 14 Gew.%).

## Patentansprüche

1. Verfahren zur Herstellung von Calciumformiat durch Umsetzung von Formaldehyd mit einer Calciumverbindung in wäßriger Phase bei einer Temperatur im Bereich von 20 bis 90 °C und Isolierung des Formiats aus der wäßrigen Phase,
dadurch gekennzeichnet,
daß man die Umsetzung in Gegenwart von Wasserstoffperoxid und/oder Calciumperoxid durchführt, wobei als Reaktionspartner für die Umsetzung Calciumoxid oder Calciumhydroxid, Formaldehyd und Wasserstoffperoxid im Molverhältnis von 1 zu 2 zu 1 bis 1,2 oder Calciumperoxid und Formaldehyd im Molverhältnis von 1 zu 2 eingesetzt werden.

2. Verfahren nach Anspruch 1,
dadurch gekennzeichnet,
daß man die Umsetzung bei einer Temperatur im Bereich von 30 bis 70 °C durchführt.

3. Verfahren nach Anspruch 1 oder 2,
dadurch gekennzeichnet,
daß man die Umsetzung zusätzlich in Gegenwart von Inhibitoren zur Inhibierung der Verzuckerung durchführt, wobei pro Mol Formaldehyd vorzugsweise 0,1 mMol bis 10 mMol Inhibitor eingesetzt werden.

4. Verfahren nach Anspruch 3,
dadurch gekennzeichnet,
daß man als Inhibitor Salze aus der Reihe von CuCl$_2$, Cu(NO$_3$)$_2$; MnCl$_2$, MnSO$_4$, Mn(II)-acetat, Mn(II)-acetylacetonat, Mn(III)-acetat; Molybdate und Wolframate der Alkali- und Eralkalimetalle; ZrCl$_4$, ZrOCl$_2$, HfCl$_4$; Borate der Alkali- und Erdalkalimetalle oder AlCl$_3$ dem Reaktionsgemisch zusetzt.

5. Verfahren nach Anspruch 3,
dadurch gekennzeichnet,
daß man die Umsetzung in Gegenwart von Inhibitoren aus der Reihe der Mangansalze, Zirkonhalogenide oder Wolframate durchführt.

6. Verfahren zur Herstellung von Calciumformiat nach einem oder mehreren der Ansprüche 1 bis 5,

dadurch gekennzeichnet,
daß man in eine Calciumhydroxid und, falls erwünscht, einen Metallsalz-Inhibitor enthaltende Suspension zunächst eine wäßrige Formaldehydlösung einträgt und anschließend unter Begrenzung der Temperatur auf maximal 60 °C eine wäßrige Wasserstoffperoxidlösung zudosiert, das Reaktionsgemisch bis zur vollständigen Umsetzung nachreagieren läßt und, sofern erforderlich, den pH-Wert mittels Ameisensäure auf 6,5 bis 7,5 stellt, unlösliche Bestandteile abtrennt und Calciumformiat durch Kristallisation aus der wäßrigen Lösung gewinnt.

**Europäisches Patentamt**

**EUROPÄISCHER RECHERCHENBERICHT**

Nummer der Anmeldung

EP 92 10 5252

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | SOVIET INVENTIONS ILLUSTRATED<br>Week 8943, 6. Dezember 1989<br>Derwent Publications Ltd., London, GB;<br>AN 89-315554 | 1,3-5 | C07C53/06 |
| D | & SU-A-1474157 ( LENNEFTEKHIM PETROC )<br>23-04-1989<br>* Zusammenfassung *<br><br>--- | | |
| A,D | 'gmelins handbuch der anorganischen chemie'<br>1956 , VERLAG CHEMIE GMBH , WEINHEIM DE<br>CALCIUM TEIL B NUMMER 28<br>* Seite 160 - Seite 162 *<br><br>----- | 1 | |
| | | | **RECHERCHIERTE SACHGEBIETE (Int. Cl.5)**<br><br>C07C |

Der vorliegende Recherchenbericht wurde für alle Patentansprüche erstellt

| Recherchenort | Abschlußdatum der Recherche | Prüfer |
|---|---|---|
| BERLIN | 29 APRIL 1992 | RUFET J. |